**Europäisches Patentamt**

**European Patent Office**

**Office européen des brevets**

(11) Publication number: **0 450 015 B1**

# EUROPEAN PATENT SPECIFICATION

(45) Date of publication of patent specification: **07.06.95**

(51) Int. Cl.⁶: **A61K 35/74**, //C12R1:21, C12R1:46,C12R1:22,C12R1:19, C12R1:445,C12R1:36

(21) Application number: **90915171.4**

(22) Date of filing: **05.10.90**

(86) International application number: **PCT/HU90/00067**

(87) International publication number: **WO 91/04742 (18.04.91 91/09)**

(54) **PHARMACEUTICAL COMPOSITION FOR THE TREATMENT OF SKIN DISEASES, ESPECIALLY PYOGENOUS AND ATOPIC DERMATITIS AND PROCESS FOR THE PREPARATION THEREOF.**

(30) Priority: **06.10.89 HU 523889**
**06.10.89 HU 523989**

(43) Date of publication of application:
**09.10.91 Bulletin  91/41**

(45) Publication of the grant of the patent:
**07.06.95 Bulletin  95/23**

(84) Designated Contracting States:
**AT BE CH DE DK ES FR GB IT LI LU NL SE**

(56) References cited:
**WO-A-87/03487**

(73) Proprietor: **SZABO, János**
**Varro u. 24**
**H-5300 Karcag (HU)**

Proprietor: **SZALAY, Laszlo**
**Vörös Hadsereg u. 5-7**
**H-5300 Karcag (HU)**

Proprietor: **SZALAY, Laszloné**

**Vörös Hadsereg u. 5-7**
**H-5300 Karcag (HU)**

(72) Inventor: **SZABO, János**
**Varro u. 24**
**H-5300 Karcag (HU)**
Inventor: **SZALAY, Laszlo**
**Vörös Hadsereg u. 5-7**
**H-5300 Karcag (HU)**
Inventor: **SZALAY, Laszloné**
**Vörös Hadsereg u. 5-7**
**H-5300 Karcag (HU)**

(74) Representative: **Sandmair, Kurt, Dr. Dr. et al**
**Patentanwälte**
**Schwabe, Sandmair, Marx**
**Postfach 86 02 45**
**D-81629 München (DE)**

## Description

TECHNICAL FIELD

The present invention relates to pharmaceutical compositions for the treatment of skin diseases, especially pyogenous and atopic dermatitis as well as a process for preparing the same. The compositions according to the invention comprise at least three of the lysates of the following bacteria: Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli and Neisseria catarrhalis.

BACKGROUND ART

Dermatitis caused by pyogenic bacteria forms a substantial part of ambulant skin diseases. Patients suffering from pyodermatitis should be regarded as infectious due to the characteristic symptoms of the disease on one hand, and the epidemyiological background on the other. Infected children should be isolated from community while adults become unable to work.

Pyogenous diseases belong to the well differentiated, classic dermatitis. Both the local and systemic treatment thereof are well-known; however, the disease is frequent and recovery frequently takes long time, while the chance for recidivation is high. In the prolonged and often recidivating cases not only the environmental susceptibility factors but also disturbances in the immunofunctions should be considered.

About 3 to 10 % of the population is suffering from or susceptible to atopic dermatitis. This polysymptomatic disease is called variously, e.g. ekzema infantum, endogenous eccema, prugiro, neurodermitis constitutionalis, etc. The disease frequently results in disappointment for both patient and doctor as the treatment hardly provides sufficient results in allergological and immunological aspects. In these cases usually a strong administration of different drugs is needed which, due to several side-effects, might be hazardous. For example, the consumption of antihistamins, antibiotics and local steroids should substantially be reduced.

In the therapy there are only a few systemic compositions and drugs, resp., wich provide an effective treatment of dermatological diseases. Most of the known compositions become effective during a very long treatment period of e.g. seven or more months and the effect is usually only transitional, accompanied by side-effects.

DISCLOSURE OF THE INVENTION

Accordingly, the present invention aims to provide a pharmaceutical composition for the treatment of the above diseases, and accordingly, to reduce the amount of other drugs used in the therapy. The composition according to the invention also aims to provide a much more effective treatment and to reduce the recidivation cases substantially.

Still another aim of the invention was to reduce the period of treatment and accordingly, the days on the sick-list.

The present invention also aims to provide a natural immunostimulant composition which is at least as active as the synthetic immunostimulant compositions used in the dermatology but which lacks of the side-effects detrimental to health.

It was surprisingly found that especially acute or chronic pyogenous or atopic dermatitis can effectively be treated with compositions comprising at least three of the lysates of the microorganisms from the group consisting of Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli and Neisseria catarrhalis.

Accordingly, the present invention relates to pharmaceutical compositions for the treatment of skin diseases, especially pyogenous and atopic dermatitis comprising at least three of the lysates of bacteria from the group consisting of Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli and Neisseria catarrhalis.

Another object of the invention is the preparation of the above compositions. These compositions are to be used in the treatment of skin diseases, especiallypyogenous and atopic dermatitis.

According to the preferred embodiment of the compositions according to the invention the lysates comprised therein are from Staphylococcus aureus, Streptococcus pyogenes and Streptococcus viridans. Of course, the scope of the active materials is not limited to the lysates of the preferred 3 bacteria; other compositions made of any cobination of 3 to 10 of the above microorganisms proved to be active as well.

A pharmaceutical composition containing the lysates of Haemophilus influenzae, Diplococcus pneumoniae, Klebsiella pneumoniae and ozaneae, Staphylococcus aureus, Streptococcus pyogenes and viridans and Neisseria catarrhalis has already been known and marketed under the name Broncho-Vaxom as 3,5 mg children's and 7 mg adult's capsules for immunostimulating the respiratory organs as immunostimulating adjuvant and for preventive therapy. However, experiments have shown that the composition did not prove to be useful in the monotherapy, for the treatment of the disease.

The Broncho-Vaxom substantially increased the number of macrophages in the mucous membrane of respiratory and digestive organs and stimulated phagocytosis. The increasing of T lymphocytes and stimulation of response of the same was also reported; this latter exceeded the response caused by synthetic immunostimulants. Moreover, the composition has a favourable influence on the T-helper/suppressor ratio.

Respiratory immunostimulation can be expected only after treatment for some weeks and months, resp., and the result shows a significance of moderate level with a certain fraction of the immunoglobulins (IgD) only. Accordingly, the composition can be used for complementary therapy only, as it has no therapeutical effect, and in the practice therapeutical effect could be detected in less than 50 % of the cases in the treatment of acute diseases (e.g. decreasing the amount of antibiotics, shorter periods of sickness, etc.) In the treatment of chronic upper respiratory catarrhous diseases no significant difference could be detected on patients treated with adjuvant and not treated, resp. (Leaflet Broncho-Vaxom, Biogal, Hungary)

It has been found that the compositions according to the present invention, including the above composition exert their effect in a strongly significant way, when used in the therapy of skin diseases, in contrary to the treatment of the respiratory tract.

Another difference from the effect on respiratory diseases lays in that the compositions are effective against the diseases already within 48 hours (e.g. on furuncle) but not later than one week (e.g. on erysipelas) in 100 % of the cases.

Accordingly, the compositions according to the present invention can be used not only in the adjuvant therapy, but also as independent therapeutica in the treatment of pyogenous and atopic dermatitis. The compositions show a strongly significant effect which can be attributed to the administration of the said composition only, and the effect on the dermatitis occurs in a substantially shorter period than in case of the respiratory diseases.

Such results could not be deducted in the knowledge of the bacterial lysates in the compositions according to the present invention. The compositions according to the invention provide a treatment for therapy which is at least equal to or usually exceeds the results of those using classic topical or systemic methods. The compositions according to the invention make also the combination with classic methods possible.

The synthetic immunomodulants intervene in the defensive mechanism in a drastical way, their effect is questionable and sometimes they can have serious side effects. The compositions according to the invention are the first dermatological immun compositions which contain natural materials, thus exerting the preferred effects of the synthetic compositions in this field, without the side effects thereof.

The quality and quantity of the bacterium lysates within the compositions may vary according to the patient, the pathogenic, the seriousness of disease, the age and state of the patient, etc.

The average daily dosis is about 0,05 to 5 mg/kg body weight, but lower and higher amounts may also be used.

BRIEF DESCRIPTION OF THE DRAWINGS

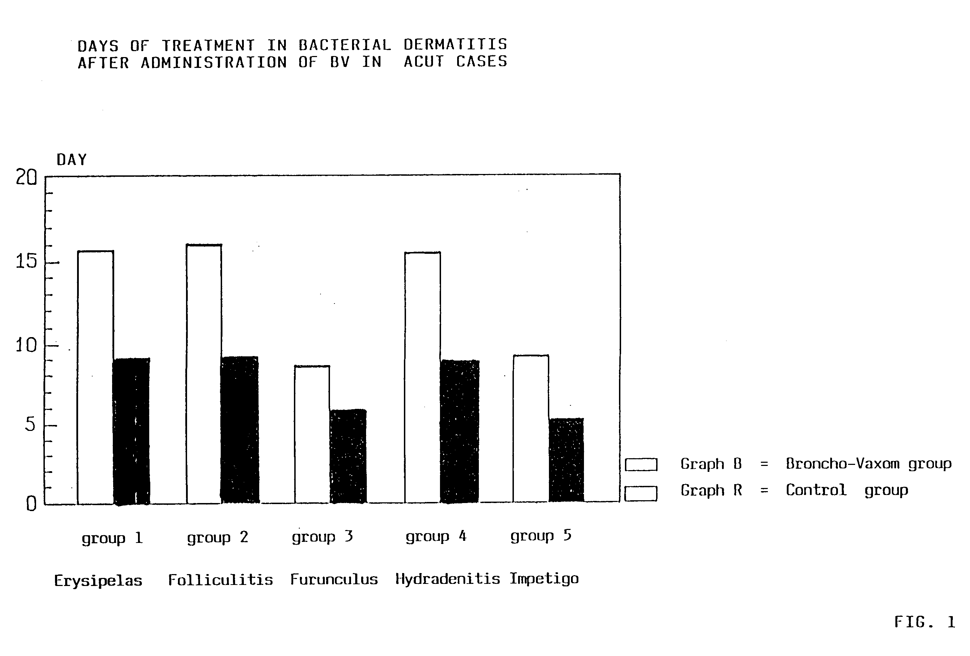

Fig 1 shows the changes due to the treatment of bacterial dermatitis with Broncho-Vaxom in acute cases.

DETAILED DESCRIPTION OF AN EMBODIMENT OF THE INVENTION

Experiments were carried out on different patients under clinical conditions to test the compositions according to the invention. Though compositions of different constitution were used for therapy, for sake of simplicity, the tests with the commercial composition Broncho-Vaxom (BV) are reported herebelow. Of course, the scope of compositions should no way be limited to the said single composition.

In the tests relating to pyodermic diseases treatment was carried out in the following pathological forms: impetigo, folliculitis, furunculus, erysipelas, hydradenitis axillaris. The admistration was made with children capsules under 14 and with adult capsules over 14. According to the disease-process the composition was administered as adjuvant therapy in 38 acute pyogenous episodes and as monotherapy for prevention in 28 cases in 4 disease groups (see Table 1).

BV was administered to a total of 116 patients, the oldest thereof being 73. The administration was performed as advised in cases of respiratory diseases (Weiss, S.: Influence of Broncho-Vaxom on serum IgE and IgG in patients with chronic obstructive disease: a double-blind controlled trial, 5th Congress of SEP, Paris, Sept. 1986).

Test I.

In acute disease tests patients were given daily 1 capsule each in the morning before meals until disappearence of symptoms, but at least 10 days. In this group, simultaneously with the administration of BV, also the routin dermatological local and/or systemic antibacterial treatment was performed. Considering the different disease-process of the above five dermatitis forms, the patient was regarded as recovered under the following circumstances:
a) at children if they returned to the children's community (nursery, school),
b) at working adults if they regained working ability,
c) at "non-working" adults if disappearence of the classic symptoms characteristic to the disease was established.
Accordingly, recovery was established according to the following criteria:
- in group furunculus: no fever, oedema and pain, necrotized follicular and perifollicular tissue emptied, epithelium unbroken;
- in group folliculitis: pustula emptied, no hyperaemic areolae, epithelium unbroken;
- in group erysipelas: no fever, pain disappeared, no oedema and complication;
- in group impetigo: no scab and oozing, epithelium-free area;
- in group hydradenitis: no pain, cutaneous and subcutaneous nodules softened, smoothened or emptied.
The period of recovery was registered in each case and compared with that registered in the year before on patients suffering from the same disease but did not receive BV. In the control group only those cases were considered wherein the first and last day of treatment was exactly registered. In this respect a total of 212 cases were considered. (see Table 2)
Statistical evaluation was made on IBM AT compatible computer system.

Test II.

In the chronic disease test patients were given each 1 capsule on each of the first 10 days of the first month and after 20 days interval, again each 1 capsule on each of the first 10 days of the second and third month in the form of intermitting treatment. In the test only adult patients were treated, wherein recidivating erysipelas, furunculosis, chronic folliculitis and hydradenitis was detected. The administration of BV was indicated according to the following criteria:
- in the erysipelas group: recidivation appeared at least 3 times in more years;
- in the group folliculitis: in spite of routin dermatological therapy forming of new pustulae was regular during at least 3 months;
- in the group furunculosis: in a period of at least 3 months more furunculus at the same time or a few, but periodically renewing furunculus were observed;
- in the group hydradenitis: in a period of at least 3 months inflammation of apocrine glands continuously appeared.
In every group the anamnesis of the patient in respect of the diagnosis for the preceding year was burdened. At these patients BV was used in the form of monotherapy to prevent recidivation. The BV treatment was started in each case wherein the patient was in remission during the routin treatment. During the intermitting treatment of 3 months the the patients were controlled 3 times, always after the 10 day's BV treatment period, and for the fourth time after an additional 3 months, during which period the patients were not administered BV. Recovery was evaluated only if no recidivation occured during the observation period.
The results of the above tests are shown on Table 3 and on Fig 1. The tests have clearly shown that the treatment period substantially decreased by the administration of BV. Compared with the average recovery period of 10,5 days of the control group, the same was 6,75 days for patients administered with BV. The difference is strongly significant in statistical evaluation (p<0,001, see Table 4). Due to the rapid improvement in the clinical symptoms, the short nursing time and consequently, decrease in the sick-list days proved the progressive character of the treatment (see Fig.1).

Fig 1. shows the preferable therapeutic effect of the BV capsule in any of the dermatitis models tested.

Test III.

The efficiency in the therapy of atopic dermatitis with the compositions according to the present invention was tested as follows:

A group of 27 patients, mostly children, suffering from atopic dermatitis were treated with BV. Administration was performed as monotherapy, combined only with a neutral ointment applied locally (Ung. hydrophylicum nonionicum). The patients were not allowed to use soap or other detergents.

BV was administered over 3 months, 10 days in one month. After each 10 day's treatment the patients were controlled.

During the treatment no side-effects were observed. Administration was performed in the winter months (December, January and February) i.e, in the period when worsening of the skin condition of patients was expected.

Except one case, symptoms of dermatitis completely regressed or substantial improvement was observed:

| | |
|---|---|
| -symptoms totally disappeared: | 19 pers. 70 % |
| -substantial improvement: | 7 pers. 26 % |
| -state unchanged: | 1 pers. 4 % |

This clinical effect can be regarded as excellent.

Our recent experiments suggested that the treat ment with the compositions according to the invention, which eliminated the symptoms of pyogenous and atopic dermatitis, resulted in a decrease of the IgE level. The reason thereof is meant probably by restoring the subpopulation of "T" secreting cells, inhibition of the histamin secretion and that of the allergic autotoxicity always being present in the atopic dermatitis. The said autotoxicity is a sort of the allergic inflammations.

Accordingly, the therapy making use of the compositions according to the invention can be used in any allergic dermatitis case, especially in cases associated with allergic inflammation, including allergic autotoxicity.

The compositions according to the invention were also succesfully used in the treatment of dermatitis other than those of pyogenous and atopis symptoms, among others:

- vasculitis nodularis,
- erythema nodosum,
- Mucha-Habermann disease, etc.

These diseases take about 50 % of the total dermatological diseases.

Table 1

| Pyodermic patients in BV therapy (in the year of 1988) | | |
|---|---|---|
| Diagnosis | No. of acute cases | No. of chronic cases |
| Erysipelas | 8 persons | 6 persons |
| Folliculitis | 13 persons | 8 persons |
| Furunculus | 46 persons | 11 persons |
| Hydradenitis | 6 persons | 3 persons |
| Impetigo | 15 persons | - |
| Total | 88 persons | 28 = 116 persons |

Table 2

| Patients in conventional therapy (Kontroll group in the year of 1986) | |
|---|---|
| Diagnosis | No. of cases |
| Erysipelas | 19 persons |
| Folliculitis | 22 persons |
| Furunculus | 63 persons |
| Hydradenitis | 6 persons |
| Impetigo | 102 persons |
| Total | 212 persons |

## Table 3

### Recovery periods

| Diagnosis | BV group | | control group | |
|---|---|---|---|---|
| | case/person | average recovery period (days) | case/person | average recovery period (days) |
| Erysipelas | 8 | $9,13 \pm 1,55$ | 19 | $15,63 \pm 4,97$ |
| Folliculitis | 13 | $9,23 \pm 2,41$ | 22 | $16 \pm 5,76$ |
| Furunculus | 46 | $5,83 \pm 1,70$ | 63 | $8,63 \pm 4,55$ |
| Hydradenitis | 6 | $8,83 \pm 1,94$ | 6 | $15,50 \pm 6,48$ |
| Impetigo | 15 | $5,33 \pm 1,40$ | 102 | $9,21 \pm 4,12$ |
| Total | 88 | $X = 6,75 \pm 1,78$ | 212 | $X = 10,50 \pm 4,56$ |

EP 0 450 015 B1

## Table 4

## Computerized statistical analysis

DAY

BY    GY

GROUP

| Source of Variation | Sum of Squares | DF | Mean Square | F | Signif of F |
|---|---|---|---|---|---|
| Main Effects | 2643.637 | 5 | 528.727 | 32.853 | 0.0 |
| GY | 996.971 | 1 | 996.971 | 61.948 | .000! |
| Group | 1771.335 | 4 | 442.834 | 27.516 | 0.0 |
| 2-way Interactions | 155.001 | 4 | 38.750 | 2.408 | .050 |
| GY Group | 155.001 | 4 | 38.750 | 2.408 | .050 |
| Explained | 2798.688 | 9 | 310.950 | 19.322 | 0.0 |
| Residual | 4667.159 | 290 | 16.094 | | |
| Total | 7465.797 | 299 | 24.969 | | |

300 (212 + 88) Cases were processed.

0           Cases (      .0 PCT) were missing.

Table 5

| Broncho-Vaxom monotherapy chronic, recidivating cases | | | |
|---|---|---|---|
| diagnosis | No. of cases | Successful | Unsuccessful |
| Erysipelas | 6 persons | 5 persons | 1 person |
| Folliculitis | 8 persons | 6 persons | 2 persons |
| Furunculus | 11 persons | 9 persons | 2 persons |
| Hydradenitis | 3 persons | 3 persons | - |
| Total | 28 persons | 23 persons | 5 persons |

## Claims

1. Pharmaceutical composition for the treatment of skin diseases, especially pyogenous and atopic dermatitis comprising at least three of the lysates of bacteria from the group consisting of Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli and Neisseria catarrhalis.

2. The composition according to claim 1 characterized by comprising the lysates from Staphyllococcus pyogenes, Streptococcus pyogenes and Streptococcus viridans.

3. Process for preparing pharmaceutical composi tion for the treatment of skin diseases, especially pyoge nous and atopic dermatitis characterized by preparing lysate from at least three of the lysates of bacteria from the group consisting of Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli and Neisseria catarrhalis and formulating the lysates thus obtained into suitable dosis form.

## Patentansprüche

1. Arzneimittel zur Behandlung von Hautkankheiten, insbesondere der pyogenen und atopischen Dermatitis, die mindestens drei Bakterienlysate aus der aus Lysaten von Haemophilus influenzae, Diplococcus pneumoniae, Klebsiella pneumoniae, K.ozaneae, Staphylococcus aureus, Streptococcus pyogenes, Str.faecalis, Str.viridans, Escherichia coli und Neisseria catarrhalis bestehenden Gruppe enthält.

2. Mittel gemäß Anspruch 1, dadurch gekennzeichnet, daß sie Lysate von Staphylococcus pyogenes, Streptococcus pyogenes und Streptococcus viridans enthält.

3. Verfahren zur Herstellung eines Arzneimittels zur Behandlung von Hautkrankheiten, insbesondere der pyogenen und atopischen Dermatitis, dadurch gekennzeichnet, daß man Lysate von mindestens drei Bakterienlysaten aus der aus Lysaten aus Haemophilus influenzae, Diplococcus pneumoniae, Klebsiella pneumoniae, K.ozaneae, Staphylococcus aureus, Streptococcus pyogenes, Str. faecalis, Str.viridans, Escherichia coli und Neisseria catarrhalis bestehenden Gruppe herstellt und die so erhaltenen Lysate zu geeigneten Dosierungsformen formuliert.

## Revendications

1. Composition pharmaceutique pour le traitement de maladies cutanées, en particulier des dermatites à pyogènes et atopiques comprenant au moins trois des lysats de bactéries du groupe constitué de Haemophilus influenzae, Diplococcus pneumoniae, Klebsiella pneumoniae, K. ozaneae, Staphylococcus aureus, Streptococcus pyogenes, Str. faecalis, Str. viridans, Escherichia coli et Neisseria catarrhalis.

2. Composition selon la revendication 1 caractérisée en ce qu'elle comprend les lysats de Staphylococcus pyogenes, Streptococcus pyogenes et Streptococcus viridans.

3. Procédé de préparation d'une composition pharmaceutique pour le traitement de maladies cutanées, en particulier des dermatites à pyogènes et atopiques caractérisé en ce qu'il comprend les étapes consistant à préparer des lysats à partir d'au moins trois des lysats de bactéries du groupe constitué de Haemophilus influenzae, Diplococcus pneumoniae, Kiebsiella pneumoniae, K. ozaneae, Staphylococcus aureus, Streptococcus pyogenes, Str. faecalis, Str. viridans, Escherichia coli et Neisseria catarrhalis et formuler les lysats ainsi obtenus sous la forme d'une dose appropriée.

DAYS OF TREATMENT IN BACTERIAL DERMATITIS
AFTER ADMINISTRATION OF BV IN  ACUT CASES

DAY

Graph B  =  Broncho-Vaxom group

Graph R  =  Control  group

group 1    group 2    group 3    group 4    group 5

Erysipelas   Folliculitis  Furunculus  Hydradenitis  Impetigo

FIG. 1